(19) Europäisches Patentamt
European Patent Office
Office européen des brevets

(11) Publication number: **0 495 554 A1**

# (12) EUROPEAN PATENT APPLICATION

(21) Application number: **92200101.1**

(22) Date of filing: **15.01.92**

(51) Int. Cl.5: **C11D 3/386**, C12N 9/42, C12N 15/56, C11D 17/06, C11D 1/62

(30) Priority: **16.01.91 EP 91870006**
**06.11.91 EP 91202881**

(43) Date of publication of application:
**22.07.92 Bulletin 92/30**

(84) Designated Contracting States:
**AT BE CH DE DK ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Convents, André Christian**
**Drielindenstraat 14**
**B-1831 Diegem(BE)**
Inventor: **Busch, Alfred**
**Habdeksstraat 210**
**B-2910 Londerzeel(BE)**
Inventor: **Baeck, André Cesar**
**Putsesteenweg 273**
**B-2820 Bonheiden(BE)**

(74) Representative: **Canonici, Jean-Jacques et al**
**Procter & Gamble European Technical Center N.V. Temselaan 100**
**B-1853 Strombeek-Bever(BE)**

## (54) Detergent compositions with high activity cellulase and quaternary ammonium compounds.

(57) The present invention provides a detergent composition comprising a quaternary ammonium compound of formula

$R_1R_2R_3R_4N^+ X^-$

wherein $R_1$ is $C_8$-$C_{16}$ alkyl, each of $R_2$, $R_3$ and $R_4$ is independently $C_1$-$C_4$ alkyl or hydroxy alkyl, benzyl, or -$(C_2H_{40})_xH$ where x has a value from 2 to 5, not more than one of $R_2$, $R_3$ or $R_4$ being benzyl, and X is an anion, and a cellulase characterized in that said cellulase provides at least 10% removal of immobilized radio-active labelled carboxymethylcellulose according to the C14 CMC-method at 25 x $10^{-6}$% by weight of cellulase protein in the laundry test solution.

According to the present invention, a preferred cellulase consists of a homogeneous endoglucanase component which is immunoreactive with a monoclonal antibody raised against a partially purified = 43 kD cellulase derived from Humicola insolens DM 1800.

EP 0 495 554 A1

## Technical field

The present invention relates to detergent compositions having cleaning and softening benefits.

## Background of the Invention

The need for detergent compositions which exhibit not only good cleaning properties, but also good fabric-softening performance, and other fabric care benefits, is well-established in the art.

EP 0 026 529 describes detergent compositions containing smectite-type clays and certain cationic compounds having cleaning and textile softening performance.

The efficiency of cellulolytic enzymes, i.e. cellulases, in terms of textile cleaning and harshness-reducing agent for fabrics has been recognized for some time; GB-A-2,075,028, GB-A-2,095,275 and GB-A-2,094,826, disclose detergent compositions with cellulase for improved cleaning performance; GB-A-1,368,599 discloses the use of cellulase for reducing the harshness of cotton-containing fabrics; U.S. 4,435,307 teaches the use of a cellulolytic enzyme derived from Humicola insolens as well as a fraction thereof, designated ACXI, as a harshness-reducing detergent additive.

EP-A-0 269 168 discloses optimized detergent compositions containing cellulase, which are formulated at a mild alkaline pH range and provide combined fabric cleaning, fabric softening, and fabric care performance.

EP-B-0 125 122 discloses a detergent composition which combines cleaning and textile softening performance by using a synergetic mixture of a long-chain tertiary amine and cellulase.

In WO 89109259 have been disclosed cellulase preparations useful for reducing the harshness of cotton-containing fabrics, comprising an endoglucanase component with a high endoase activity and affinity towards cellulose.

The practical exploitation of cellulases has however, been set back by the fact that cellulase preparations such as those disclosed in the above-mentioned prior art documents, are complex mixtures, of which only a certain fraction is effective in the fabric-care context; it was thus difficult to implement cost effective industrial production of cellulase for the detergent industry; and large quantities of such cellulase preparations would need to be applied, in order to obtain the desired effect on fabrics.

Improvements in cellulase production also often have not proven to be sufficiently identifiable in terms of applicability in detergents. Defining a cellulase selection criterium relevant for detergent application of cellulase was made possible by the C14CMC-method disclosed in EP-A-350 098. A minimum of 10% removal of immobilized radioactive labelled carboxymethylcellulose at $25 \times 10^{-6}$% by weight of cellulase protein in the laundry test solution has been found to provide high activity cellulase. A preferred group of cellulase falling under the high activity definition according to the present invention has been disclosed in copending Danish Patent Application No. : 1159/90 filed May 5, 1990. There is disclosed a cellulase preparation consisting essentially of a homogeneous endoglucanase component which is immunoreactive with a monoclonal antibody raised against a partially purified 43kD cellulase derived from Humicola insolens DM1800.

The finding that this particular endoglucanase component of cellulase is advantageous for the treatment of cellulose-containing materials now permits to produce the cellulase cost-effectively, e.g. by employing recombinant DNA techniques, and allows to apply only a small quantity of the cellulase preparation, and obtain the desired effect on fabrics.

It has surprisingly been found that an improved detergent composition can be formulated which combine superior cleaning and softening performance by using a synergestic mixture of water soluble quaternary ammonium compounds and high active cellulase. Said cellulase having at least 10% CMC removal at $25 \times 10^{-6}$% by weight of cellulase protein in the laundry test solution.

It is another object of the present invention to provide a method for treating fabrics in a washing machine, comprising the utilization of the present detergent compositions, for the main wash cycle.

## Summary of the Invention

The present invention provides a detergent composition comprising a quaternary ammonium compound of formula

$$R_1 R_2 R_3 R_4 N^+ X^-$$

wherein $R_1$ is $C_8$-$C_{16}$ alkyl, each of $R_2$, $R_3$ and $R_4$ is independently $C_1$-$C_4$ alkyl or hydroxy alkyl, benzyl, or

- $(C_2H_4O)_xH$ where x has a value from 2 to 5, not more than one of $R_2$, $R_3$ or $R_4$ being benzyl, and X is an anion, and a cellulase characterized in that said cellulase provides at least 10% removal of immobilized radio-active labelled carboxymethylcellulose according to the C14 CMC-method at 25 x $10^{-6}$% by weight of cellulase protein in the laundry test solution.

According to the present invention, a preferred cellulase consists of a homogeneous endoglucanase component which is immunoreactive with a monoclonal antibody raised against a partially purified = 43 kD cellulase derived from Humicola insolens DM 1800.

## Detailed Description of the Invention

### CELLULASE

The activity of enzymes and particularly the activity of cellulase enzyme has been defined for various applications by different analytical methods. These methods all attempt to provide a realistic assessment of the expected in use performance or at least a measurement correlating with the in use performance. As has been detailed in European Patent Application EP-A-350098, many of the methods, particularly these frequently used by cellulase manufacturers, are not sufficiently correlated with the in use performance of cellulase in laundry detergent compositions. This is due to the various other usage conditions for which these activity measurement methods have been developed.

The method described in EP-A-350098, has been developed to be and to have a predictive correlation for the ranking of cellulase activity in laundry detergent compositions.

The present invention therefore uses the method disclosed in EP-A-350098 to screen cellulases in order to distinguish cellulases which are useful in the present invention and those which would not provide the objectives of the present invention. The screening method, hereinafter referred to as C14CMC-Method, which has been adopted from the method disclosed in EP-A-350098, can be described as follows :

### Principle :

The principle of the C14CMC-Method for screening is to measure at a defined cellulase concentration in a wash solution the removal of immobilized carboxy methyl cellulose (CMC) from a cloth substrate. The removal of CMC is measured by radio-active labelling of some of the CMC by using C14 radio-active carbon. Simple counting of the amount of radio-active C14 on the cloth substrate before and after the cellulase treatment allows the evaluation of the cellulase activity.

### Sample preparation :

**CMC preparation :** The radio-active CMC stock solution is prepared according to Table I. The radio-active CMC can be obtained by methods referred to in EP-A-350098.

**Fabric substrates :** The fabric substrates are muslin cotton swatches having a size of 5 cm x 5 cm. They are inocculated with 0.35 ml of the radio-active labelled CMC stock solution in their center. The muslin cottonswatches are then airdried.

**Immobilization of CMC :** To immobilize the radio-active labelled CMC on the muslin cotton swatches, laundero-meter equipment " Linitest Original Haunau " made by Original Haunau, Germany, is used. A metal jar of the laundero-meter is filled with 400 ml of hard water (4 mmol/liter of $Ca^{++}$ ions). A maximum number of 13 swatches can be used per jar. The jar is then incubated in a heat-up cycle from 20 °C to 60 °C over 40 minutes in the laundero-meter equipment. After incubation the swatches are rinsed under running city water for 1 minute. They are squeezed and allowed to airdry for at least 30 minutes.

According to EP-A-350098 samples of the swatches with immobilized radio-active CMC can also be measured as "blank samples" without washing.

### Sample treatment :

**Laundry test solution :** The laundry test solution is prepared according to the composition of Table II. It is balanced to pH 7.5. The laundry test solution is the basis to which a cellulase test sample is added. Care should be taken to not dilute the laundry test solution by adding water to a 100% balance prior to having determined the amount of cellulase to be added. The amount of cellulase which is used in this screening test should be added to provide 25 x $10^{-6}$ weight percent of cellulase protein in the laundry test solution (equivalent to 0.25 milligram/liter at 14.5 °C).

**Wash procedure :** The swatches thus inocculated with radio-active labelled CMC are then treated in a laundry simulation process. The laundry process is simulated in the laundero-meter type equipment," Linitest, Original Haunau", by Original Haunau, Haunau Germany. An individual swatch is put into a 20 $cm^3$ glass vial. The vial is filled with 10 ml of the laundry test solution and then sealed liquid tight. Up to 5 vials are put into each laundero-meter jar. The jar is filled with water as a heat tranfer medium for the laundering simulation. The laundering simulation is conducted as a heat-up cycle from 20°C to 60°C over 40 minutes.

After the processing of the samples the vials are submerged in cold water and subsequently each swatch is taken out of its vial, rinsed in a beaker under running soft water, squeezed and allowed to airdry for at least 30 minutes.

Measurement :

In order to measure radio-active labelled CMC removal, a scintillation counter, for example, a LKB 1210 Ultrabeta Scintillation Counter, is used. In order to obtain most accurate results, the instruction manual for optimum operation of the particular scintillation counter should be followed. For example, for the LKB 1210 Ultrabeta Scintillation Counter, the following procedure should be followed. The swatch to be measured is put into a plastic vial filled with 12 ml of scintillator liquid (e.g. scintillator 299 from Packard). The swatch is then allowed to stabilize for at least 30 minutes. The vial is then put into the LKB 1210 Ultrabeta Scintillation Counter and the respective radio-activity counts for the swatch is obtained.

In order to measure the amount of CMC removal due only to the cellulase, a measurement of a swatch which has been inocculated at the same time but has been treated in the laundry test solution without cellulase, is necessary. The activity of the cellulase is then expressed as percent of radio-active labelled CMC removal. This percentage is calculated by the following formula :

$$\text{\% of radio-active CMC removal} = \frac{XO - XC}{XO} \times 100$$

Wherein

XO is the radioactivity scintillation count of a swatch treated with the laundry test solution without cellulase

XC is the radioactivity scintillation count of a swatch treated with the laundry test solution containing the cellulase to be evaluated

**Statistical considerations, procedure confirmation :**

In order to provide statistically sound results, standard statistical analysis should be employed. For the given example, using the LKB 1210 Ultrabeta Scintillation Counter, it has been found that a sample size of 3 swatches for each radioactivity scintillation count can be used.

In order to confirm the procedure by internal crosschecking, measurement and calculation of the "blank sample" according to EP-A-350098 are recommended. This will allow to detect and eliminate errors.

Interpretation of results :

The described screening test does provide a fast, unique and reliable method to identify cellulases which satisfy the activity criteria of the present invention versus cellulases which are not part of the present invention.

It has been found that a removal of 10% or more of the immobilized radioactive labelled CMC according to the above C14CMC-method, indicates that the respective cellulase satisfies the requirements of the invention.

It will be obvious to those skilled in the art that removal percentages above 10% indicate a higher activity for the respective cellulase. It therefore is contemplated that cellulase providing above 25% or preferably above 50% removal of radioactive labelled CMC, at the protein concentration in the laundry test solution according to the C14CMC-method, would provide indication of an even better performance of the cellulase for use in laundry detergents.

It also has been contemplated that usage of higher concentrations of cellulase for C14CMC-method, would provide higher removal percentages. However, there exists no linear proven correlation between

cellulase concentration and removal percentage obtained by it.

It also has been contemplated that usage of higher concentrations of cellulase for C14CMC-method, would provide higher removal percentages.

TABLE I

| Radioactive $C_{14}$ labelled CMC stock solution (all percentages by weight of total solution) | |
|---|---|
| Total CMC* (CMC should be detergent grade CMC with a degree of substitution from about 0.47 to about 0.7) | $99.2 \times 10^{-3}$% |
| Ethanol | $14985.12 \times 10^{-3}$% |
| Deionized Water | $84915.68 \times 10^{-3}$% |
| Total : | 100% |

* Total CMC contains non-radio-active and radio-active CMC to provide a radio-activity which allows sufficiently clear readings on the scintillation counter used. For example, the radio-active CMC can have an activity of 0.7 millicurie/g and be mixed with non-radio-active CMC at a ratio of 1:6.7.

TABLE II

| Laundry test solution (all percentages by weight of total solution) | |
|---|---|
| Linear $C_{12}$ alkyl benzene sulphonic acid | 0.110% |
| Coconut alkyl sulphate (TEA salt) | 0.040% |
| $C_{12-15}$ alcohol ethoxylate (E07) | 0.100% |
| Coconut fatty acid | 0.100% |
| Oleic acid | 0.050% |
| Citric acid | 0.010% |
| Triethanolamine | 0.040% |
| Ethanol | 0.060% |
| Propanediol | 0.015% |
| Sodium hydroxide | 0.030% |
| Sodium formate | 0.010% |
| Protease | 0.006% |
| Water (2.5 mmol/liter $Ca^{++}$), pH adjustment agent (HCL or NaOH solutions) and cellulase | balance to 100% |

According to the present invention, preferred cellulases are those as described in Danish Patent Application 1159/90. For example, a cellulase preparation useful in the compositions of the invention can consist essentially of a homogeneous endoglucanase component, which is immunoreactive with an antibody raised against a highly purified 43kD cellulase derived from Humicola insolens, DSM 1800, or which is homologous to said 43kD endoglucanase.

It should be stressed that all cellulase enzymes according to the present invention have to meet the criteria of the above mentioned screening test. However, in the Danish Patent Application 1159/90 additional criteria are established allowing to identify preferred cellulase enzymes in combination with the present screening test.

Cellulase preparations particularly useful in the compositions of the invention are those in which in

addition to the screening test, the endoglucanase component exhibits a CMC-endoase activity of at least about 50, preferably at least about 60, in particular at least about 90 CMC-endoase units per mg of total protein. In particular, a preferred endoglucanase component exhibits a CMC-endoase activity of at least 100 CMC-endoase units per mg of total protein.

In the present context, the term "CMC-endoase activity" refers to the endoglucanase activity of the endoglucanase component in terms of its ability to degrade cellulose to glucose, cellobiose and triose, as determined by a viscosity decrease of a solution of carboxymethyl cellulose (CMC) after incubation with the cellulase preparation of the invention, as described in detail below.

The CMC-endoase (endoglucanase) activity can be determined from the viscosity decrease of CMC, as follows : A substrate solution is prepared, containing 35 g/l CMC (Hercules 7 LFD) in 0.1 M tris buffer at pH 9.0. The enzyme sample to be analyzed is dissolved in the same buffer. 10 ml substrate solution and 0.5 ml enzyme solution are mixed and transferred to a viscosimeter (e.g. Haake VT 181, NV sensor, 181 rpm), thermostated at 40°C. Viscosity readings are taken as soon as possible after mixing and again 30 minutes later. The amount of enzyme that reduces the viscosity to one half under these conditions is defined as 1 unit of CMC-endoase activity, or CEVU/liter.

SDS polyacrylamide gel electrophoresis (SDS-PAGE) and isoelectric focusing with marker proteins in a manner known to persons skilled in the art were used to determine the molecular weight and isolelectric point (pI), respectively, of the endoglucanase component in the cellulase preparation useful in the present context. In this way, the molecular weight of a specific endoglucanase component was determined to be 43kD. The isoelectric point of this endoglucanase was determined to be about 5.1.

The cellobiohydrolase activity may be defined as the activity towards cellobiose p-nitrophenyl. The activity is determined as $10^{-6}$ mole nitrophenyl released per minute at 37°C and pH 7.0. The present endoglucanase component was found to have essentially no cellobiohydrolase activity.

The endoglucanase component in the cellulase preparation herein has initially been isolated by extensive purification procedures, i.a. involving reverse phase HPLC purification of a crude H. insolens cellulase mixture according to U.S. 4,435,307. This procedure has surprisingly resulted in the isolation of a 43kD endoglucanase as a single component with unexpectedly favourable properties due to a surprisingly high endoglucanase activity.

Also, in addition to the screening test, the cellulase enzymes useful in the present compositions can further be defined as enzymes exhibiting endoglucanase activity (in the following referred to as an "endoglucanase enzyme"), which enzymes have the amino acid sequence shown in the appended Sequence Listing ID#2, or a homologue thereof exhibiting endoglucanase activity.

In the present context, the term "homologue" is intended to indicate a polypeptide encoded by DNA which hybridizes to the same probe as the DNA coding for the endoglucanase enzyme with this amino acid sequence under certain specified conditions (such as presoaking in 5xSSC and prehybridizing for 1 h at 40°C in a solution of 20% formamide, 5xDenhardt's solution, 50 mM sodium phosphate, pH 6.8, and 50 ug of denatured sonicated calf thymus DNA, followed by hybridization in the same solution supplemented with 100 uM ATP for 18 h at 40°C). The term is intended to include derivatives of the aforementioned sequence obtained by addition of one or more amino acid residues to either or both the C- and N-terminal of the native sequence, substitution of one or more amino acid residues at one or more sites in the native sequence, deletion of one or more amino acid residues at either or both ends of the native amino acid sequence or at one or more sites within the native sequence, or insertion of one or more amino acid residues at one or more sites in the native sequence.

The endoglucanase enzyme herein may be one producible by species of Humicola such as Humicola insolens e.g. strain DSM 1800, deposited on October 1, 1981 at the Deutsche Sammlung von Mikroorganismen, Mascheroder Weg 1B, D-3300 Braunschweig, FRG, in accordance with the provisions of the Budapest Treaty on the International Recognition of the Deposit of Microorganisms for the Purposes of Patent Procedure (the Budapest Treaty).

In still a further aspect, the cellulase enzymes useful herein can be defined, in addition to the screening test, as endoglucanase enzymes which have the amino acid sequence shown in the appended Sequence Listing ID#4, or a homologue thereof (as defined above) exhibiting endoglucanase activity. Said endoglucanase enzyme may be one producible by a species of Fusarium, such as Fusarium oxysporum, e.g. strain DSM 2672, deposited on June 6, 1983 at the Deutsche Sammlung von Mikroorganismen, Mascheroder Weg 1B, D-3300 Braunschweig, FRG, in accordance with the provisions of the Budapest Treaty.

Furthermore, it is contemplated that homologous endoglucanases may be derived from other microorganisms producing cellulolytic enzymes, e.g. species of Trichoderma, Myceliophthora, Phanerochaete, Schizophyllum, Penicillium, Aspergillus, and Geotricum.

For industrial production of the cellulase preparation herein, however, it is preferred to employ recombinant DNA techniques or other techniques involving adjustements of fermentations or mutation of the microorganisms involved to ensure overproduction of the desired enzymatic activities. Such methods and techniques are known in the art and may readily be carried out by persons skilled in the art.

The endoglucanase component may thus be one which is producible by a method comprising cultivating a host cell transformed with a recombinant DNA vector which carries a DNA sequence encoding said endoglucanase component or a precursor of said endoglucanase component as well as DNA sequences encoding functions permitting the expression of the DNA sequence encoding the endoglucanase component or precursor thereof, in a culture medium under conditions permitting the expression of the endoglucanase component or precursor thereof and recovering the endoglucanase component from the culture.

DNA constructs comprising a DNA sequence encoding an endoglucanase enzyme as described above, or a precursor form of the enzyme, include the DNA constructs having a DNA sequence as shown in the appended Sequence Listings ID#1 or ID#3, or a modification thereof. Examples of suitable mofidications of the DNA sequence are nucleotide substitutions which do not give rise to another amino acid sequence of the endoglucanase, but which correspond to the codon usage of the host organism into which the DNA construct is introduced or nucleotide substitutions which do give rise to a different amino acid sequence and therefore, possibly, a different protein structure which might give rise to an endoglucanase mutant with different properties than the native enzyme. Other examples of possible modifications are insertion of one or more nucleotides at either end of the sequence, or deletion of one or more nucleotides at either end or within the sequence.

DNA constructs encoding endoglucanase enzymes useful herein may be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, annealed, ligated and cloned in suitable vectors.

A DNA construct encoding the endoglucanase enzyme or a precursor thereof may, for instance, be isolated by establishing a cDNA or genomic library of a cellulase-producing microorganism, such as Humicola insolens, DSM 1800, and screening for positive clones by conventional procedures such as by hybridization using oligonucleotide probes sythesized on the basis of the full or partial amino acid sequence of the endoglucanase in accordance with standard techniques (cf. Sambrook et al, Molecular Cloning : A Laboratory Manual, 2nd Ed. Cold Spring Harbor, 1989), or by selecting for clones expressing the appropriate enzyme activity (i.e. CMC-endoase activity as defined above), or by selecting for clones producing a protein which is reactive with an anti-body against a native cellulase (endoglucanase)

Finally, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques. The DNA construct may also be prepared by polymerase chain reaction using specific primers, for instance as described in US 4,683,202 or R. K. Saiki et al, Science 239, 1988, pp. 487-491.

Recombinant expression vectors into which the above DNA constructs are inserted include any vector which may conveniently be subjected to recombinant DNA procedures, and the choice of vector will often depend on the host cell into which it is to be introduced. Thus, the vector may be an autonomously replicating vector, i.e. a vector which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into wich it has been integrated.

In the vector, the DNA sequence encoding the endoglucanase should be operably connected to a suitable promoter and terminator sequence. The promoter may be any DNA sequence which shows transcriptional activity in the host cell of choice and may be derived from genes encoding proteins either homologous or heterologous to the host cell. The procedures used to ligate the DNA sequences coding for the endoglucanase, the promoter and the terminator, respectively, and to insert them into suitable vectors are well known to persons skilled in the art (cf., for instance, Sambrook et al., op.cit.).

Host cells which are transformed with the above DNA constructs or the above expression vectors may be for instance belong to a species of Aspergillus, most preferably Aspergillys oryzae or Aspergillus niger. Fungal cells may be transformed by a process involving protoplast formation and transformation of the protoplasts followed by regeneration of the cell wall in a manner known per se. The use of Aspergillus as a host microorganism is described in EP 238 023 (of Novo Industri A/S), the contents of which are hereby incorporated by reference. The host cell may also be a yeast cell, e.g. a strain of Saccharomyces

cerevisiae.

Alternatively, the host organism may be a bacterium, in particular strains of Streptomyces and Bacillus, and E. coli. The transformation of bacterial cells may be performed according to conventional methods, e.g. as described in Sambrook et al., Molecular Cloning: A Laboratory Manual, Cold Spring Harbor, 1989.

The screening of appropriate DNA sequences and construction of vectors may also be carried out by standard procedures, cf. Sambrook et al., op.cit.

The medium used to cultivate the transformed host cells may be any conventional medium suitable for growing the host cells in question. The expressed endoglucanase may conveniently be secreted into the culture medium and may be recovered therefrom by well-known procedures including separating the cells from the medium by centrifugation or filtration, precipitating proteinaceous components of the medium by means of a salt such as ammonium sulphate, followed by chromatographic procedures such as ion exchange chromatography, affinity chromatography, or the like.

By employing recombinant DNA techniques as indicated above, techniques of protein purification, techniques of fermentation and mutation or other techniques which are well known in the art, it is possible to provide endoglucanases of a high purity.

The level in the present composition of cellulase described above should be such that the amount of enzyme protein to be delivered in the wash solution is from 0.005 to 40 mg/liter of wash solution, preferably 0.01 to 10 mg/liter of wash solution.

The water-soluble quaternary ammonium compound

The water-soluble soluble quaternary ammonium compounds useful in the present composition have the formula :

$$R_1R_2R_3R_4N^+X^-$$

wherein $R_1$ is $C_8$-$C_{16}$ alkyl, each of $R_2$, $R_3$ and $R_4$ is independently $C_1$-$C_4$ alkyl, $C_1$-$C_4$ hydroxy alkyl, benzyl, and $-(C_2H_{40})_xH$ where x has a value from 2 to 5, and X is an anion. Not more than one of $R_2$, $R_3$ or $R_4$ should be benzyl.

The preferred alkyl chain length for $R_1$ is $C_{12}$-$C_{15}$ particularly where the alkyl group is a mixture of chain lengths derived from coconut or palm kernel fat or is derived synthetically by olefin build up or OXO alcohols synthesis. Preferred groups for $R_2R_3$ and $R_4$ are methyl and hydroxyethyl groups and the anion X may be selected from halide, methosulphate, acetate and phosphate ions. Examples of suitable quaternary ammonium compounds for use herein are

coconut trimethyl ammonium chloride or bromide
coconut methyl dihydroxyethyl ammonium chloride or bromide
decyl triethyl ammonium chloride or bromide
decyl dimethyl hydroxyethyl ammonium chloride or bromide
C12-14 dimethyl hydroxyethyl ammonium chloride or bromide
myristyl trimethyl ammonium methyl sulphate
lauryl dimethyl benzyl ammonium chloride or bromide
lauryl methyl (ethenoxy)$_4$ ammonium chloride or bromide

The above water-soluble cationic components of the compositions of the present invention, are capable of existing in cationic form in a 0.1% aqueous solution at pH 10.

The water-soluble cationic compounds are present in an amount of from 0.2% to 10% by weight of the detergent compositions.

DETERGENT ADJUNCTS

A wide range of surfactants can be used in the detergent compositions. A typical listing of anionic, nonionic, ampholytic and zwitterionic classes, and species of these surfactants, is given in US Patent 3,664,961 issued to Norris on May 23, 1972.

Mixtures of anionic surfactants are particularly suitable herein, especially mixtures of sulphonate and sulphate surfactants in a weight ratio of from 5:1 to 1:2, preferably from 3:1 to 2:3, more preferably from 3:1 to 1:1. Preferred sulphonates include alkyl benzene sulphonates having from 9 to 15, especially 11 to 13 carbon atoms in the alkyl radical, and alpha-sulphonated methyl fatty acid esters in which the fatty acid is derived from a $C_{12}$-$C_{18}$ fatty source preferably from a $C_{16}$-$C_{18}$ fatty source. In each instance the cation is an alkali metal, preferably sodium. Preferred sulphate surfactants are alkyl sulphates having from 12 to 18

carbon atoms in the alkyl radical, optionally in admixture with ethoxy sulphates having from 10 to 20, preferably 10 to 16 carbon atoms in the alkyl radical and an average degree of ethoxylation of 1 to 6. Examples of preferred alkyl sulphates herein are tallow alkyl sulphate, coconut alkyl sulphate, and $C_{14-15}$ alkyl sulphates. The cation in each instance is again an alkali metal cation, preferably sodium.

One class of nonionic surfactants useful in the present invention are condensates of ethylene oxide with a hydrophobic moiety to provide a surfactant having an average hydrophilic-lipophilic balance (HLB) in the range from 8 to 17, preferably from 9.5 to 13.5, more preferably from 10 to 12.5. The hydrophobic (lipophilic) moiety may be aliphatic or aromatic in nature and the length of the polyoxyethylene group which is condensed with any particular hydrophobic group can be readily adjusted to yield a water-soluble compound having the desired degree of balance between hydrophilic and hydrophobic elements.

Especially preferred nonionic surfactants of this type are the $C_9$-$C_{15}$ primary alcohol ethoxylates containing 3-8 moles of ethylene oxide per mole of alcohol, particularly the $C_{14}$-$C_{15}$ primary alcohols containing 6-8 moles of ethylene oxide per mole of alcohol and the $C_{12}$-$C_{14}$ primary alcohols containing 3-5 moles of ethylene oxide per mole of alcohol.

Another class of nonionic surfactants comprises alkyl polyglucoside compounds of general formula

$RO\ (C_nH_{2n}O)_tZ_x$

wherein Z is a moiety derived from glucose; R is a saturated hydrophobic alkyl group that contains from 12 to 18 carbon atoms; t is from 0 to 10 and n is 2 or 3; x is from 1.3 to 4, the compounds including less than 10% unreacted fatty alcohol and less than 50% short chain alkyl polyglucosides. Compounds of this type and their use in detergent are disclosed in EP-B 0 070 077, 0 075 996 and 0 094 118.

Also suitable as nonionic surfactants are poly hydroxy fatty acid amide surfactants of the formula

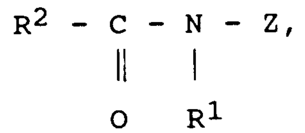

wherein $R^1$ is H, or $R^1$ is $C_{1-4}$ hydrocarbyl, 2-hydroxy ethyl, 2-hydroxy propyl or a mixture thereof, $R^2$ is $C_{5-31}$ hydrocarbyl, and Z is a polyhydroxyhydrocarbyl having a linear hydrocarbyl chain with at least 3 hydroxyls directly connected to the chain, or an alkoxylated derivative thereof. Preferably, $R^1$ is methyl, $R^2$ is a straight $C_{11-15}$ alkyl or alkenyl chain such as coconut alkyl or mixtures thereof, and Z is derived from a reducing sugar such as glucose, fructose, maltose, lactose, in a reductive amination reaction.

The compositions according to the present invention may further comprise a builder system. Any conventional builder system is suitable for use herein including aluminosilicate materials, silicates, polycarboxylates and fatty acids, materials such as ethylenediamine tetraacetate, metal ion sequestrants such as aminopolyphosphonates, particularly ethylenediamine tetramethylene phosphonic acid and diethylene triamine pentamethylenephosphonic acid. Though less preferred for obvious environmental reasons, phosphate builders can also be used herein.

Suitable builders can be an inorganic ion exchange material, commonly an inorganic hydrated aluminosilicate material, more particularly a hydrated synthetic zeolite such as hydrated zeolite A, X, B or HS.

Another suitable inorganic builder material is layered silicate, e.g. SKS-6 (Hoechst). SKS-6 is a crystalline layered silicate consisting of sodium silicate ($Na_2Si_2O_5$).

Suitable polycarboxylates builders for use herein include citric acid, preferably in the form of a water-soluble salt, derivatives of succinic acid of the formula R-CH(COOH)CH2(COOH) wherein R is C10-20 alkyl or alkenyl, preferably C12-16, or wherein R can be substituted with hydroxyl, sulfo sulfoxyl or sulfone substituents. Specific examples include lauryl succinate , myristyl succinate, palmityl succinate-2-dodecenylsuccinate, 2-tetradecenyl succinate. Succinate builders are preferably used in the form of their water-soluble salts, including sodium, potassium, ammonium and alkanolammonium salts.

Other suitable polycarboxylates are oxodisuccinates and mixtures of tartrate monosuccinic and tartrate disuccinic acid such as described in US 4,663,071.

Especially for the liquid execution herein, suitable fatty acid builders for use herein are saturated or unsaturated C10-18 fatty acids, as well as well as the corresponding soaps. Preferred saturated species have from 12 to 16 carbon atoms in the alkyl chain. The preferred unsaturated fatty acid is oleic acid. Preferred builder systems for use in granular compositions include a mixture of a water-insoluble aluminosilicate builder such as zeolite A, and a watersoluble carboxylate chelating agent such as citric acid.

Other builder materials that can form part of the builder system for use in granular compositions the purposes of the invention include inorganic materials such as alkali metal carbonates, bicarbonates, silicates, and organic materials such as the organic phosphonates, amiono polyalkylene phosphonates and amino polycarboxylates.
Other suitable water-soluble organic salts are the homo- or copolymeric acids or their salts, in which the polycarboxylic acid comprises at least two carboxyl radicals separated from each other by not more than two carbon atoms.
Polymers of this type are disclosed in GB-A-1,596,756. Examples of such salts are polyacrylates of MW 2000-5000 and their copolymers with maleic anhydride, such copolymers having a molecular weight of from 20,000 to 70,000, especially about 40,000.

Detergency builder salts are normally included in amounts of from 10% to 80% by weight of the composition preferably from 20% to 70% and most usually from 30% to 60% by weight.

Bleaching agents, suds controlling agents, soil suspending agents, proteolytic, amylolytic or lipolytic enzymes, especially proteolytic, and optical brighteners, may be present.

Colours, non-substantive, and perfumes, as required to improve the aesthetic acceptability of the product, are usually incorporated.

The detergent compositions according to the invention can be in liquid, paste or granular forms. Granular compositions according to the present invention can also be in "compact form", i.e. they may have a relatively higher density than conventional granular detergent compositions according to the present invention will contain a lower amount of "inorganic filler salt", compared to conventional granular detergents; typical filler salts are alkaline earth metal salts of sulphates and chlorides, typically sodium sulphate; "compact" detergents typically comprise not more than 10% filler salt.

Preparation of the compositions

The detergent compositions may be prepared in any way, as appropriate to their physical form, as by mixing the components, co-agglomerating them or dispersing them in a liquid carrier. In granular form a detergent salt builder can be incorporated and the granular is prepared by spray drying an aqueous slurry of the non-heat-sensitive components, and the builder salt to form spray dried granules into which may be admixed the heat sensitive components such as persalts, enzymes, perfumes. The water-soluble quaternary compound may be included in the slurry for spray drying or it may be incorporated by dissolving or dispersing the cationic component in water or another suitable volatile liquid and then spraying this solution of disperion onto the spray dried granules before or after other heat sensitive solids have been dry mixed with them. Alternatively the water-soluble quaternary compound can be dry mixed together with the other heat sensitive solids. Clay components may be added to the slurry for spray drying or may be dry mixed, as preferred for reasons unrelated to its softening effect, such as for optimum colour of the product.

The invention is illustrated by the following non-limiting examples.

The following examples are meant to exemplify compositions of the present invention, but are not necessarily meant to limit or otherwise define the scope of the invention, said scope being determined according to claims which follow.

EXAMPLE I

Criticality of the cellulase performance parameter of claim 1

The following test was conducted :

**Test conditions :**

Washing temperature : 60 ° C (heat up cycle)
Washing time : 40 min.
pH = 7.5
Water hardness : 4 mmol/L
Detergent concentration : 1%
Detergent composition : crf. EPA 350 098 ex. 1
Cellulases :
1) Celluzyme$^R$ supplied by Novo Nordisk = reference
2) 43kD endoglucanase = cellulase according to the invention

**Test Results :**

| | % C14-CMC Removal by Cellulase |
|---|---|
| Detergent without cellulase (= reference) | 0 |
| Detergent + Celluzyme[R] | |
| 1.5 mg protein/L ($150 \times 10^{-6}$%) | 12.7 |
| 3.0 mg protein/L ($300 \times 10^{-6}$%) | 17.7 |
| 4.5 mg protein/L ($450 \times 10^{-6}$%) | 21.5 |
| Detergent + 43kD endoglucanase | |
| 0.3 mg protein/L ($30 \times 10^{-6}$%) | 20.3 |

**Discussion of the results :**

The above data clearly demonstrate the criticality of the claimed parameter for the cellulases of the invention over the commercially available Celluzyme.

EXAMPLE II

Two sets of different detergent compositions are prepared, all based on a compact granular detergent composition.
Such a compact granular detergent composition typically contains the following ingredients :

| | |
|---|---|
| Linear alkyl benzene sulphonate (LAS) | 9.5% |
| Alkyl suphate | 3% |
| Nonionic | 4% |
| Trisodium citrate | 21% |
| Zeolite | 33% |
| Citric acid | 6% |
| Polymer | 4% |
| Chelant | 0.2% |
| Sodium sulphate | 6% |
| Sodium silicate | 2% |
| Perborate | 0.5% |
| Phenol sulphonate | 0.1% |

The above detergent composition was supplemented as indicated below :

SET 1 : With 43kD endoglucanase

| (*)Quaternary ammonium compounds level mg/l | No Cellulase (PSU) | 2.4 mg Cellulase/L wash liquor ($240 \times 10^{-6}$ %) (PSU) |
|---|---|---|
| 0 | -2.5 | 0 |
| 100 | -2.5 | 1.5 |
| 200 | -2.5 | 3.5 |

(*) $C_{12}$-$C_{14}$ dimethyl (hydroxyethyl) ammonium chloride

**SET 2 : With Celluzyme$^R$**

| (*)Quaternary ammonium compounds level mg/l | No Cellulase (PSU) | 92 mg Cellulase/L wash liquor ($9200 \times 10^{-6}$ %) (PSU) |
|---|---|---|
| 0 | -0.3 | 0 |
| 100 | -0.3 | 0 |
| 200 | -0.3 | 0 |

(*) $C_{12}$-$C_{14}$ dimethyl (hydroxyethyl) ammonium chloride

Test procedure :

Cellulase has the property to de-pill worn cotton fabrics. In a model test the measurement of de-pilling is used to assess cellulase performance.
Swatches of worn blue pyjama fabric were treated with different wash solutions in a Laundrometer (temperature 30° C). The water hardness was 2.5 mM Calcium. After tumble drying, the fabrics were graded for de-pilling by direct comparison of the different detergent matrices. Visual grading was performed by expert judges using a 0 to 4 scale (PSU). In this scale 0 is fiven for no difference and 4 is given for maximum difference. The PSU grading are statistical recount, an average of 4 replicates is made, LSD (least significant difference) is 0.5 PSU at 95% confidence level.
The above results demonstrate the synergy between the quaternary ammonium compound and the 43 kD cellulase in that the performance of the 43 kD cellulase is significantly improved by the quaternary ammonium compound.

EXAMPLE III-VI

Test procedure :

3.5 kg of clean fabric laundry loads were washed in an automatic drum washing machine Miele 423 at 60° C. The hardness of the water was 2,5 mM Calcium and the composition concentration was 0.7% in the wash liquor. For softness evaluation swatches of terry towel were line dried prior for assessment of softness. Comparative softness assessment was done by expert judges using a scale of 0 to 4 panel-score-units (PSU). In this scale 0 is given for no difference and 4 is given for maximum difference. Softness was assessed after one and after one, four and eight wash cycles. LSD is 0.5 psu at the 95% confidence level.
The following compositions are made :

| Ingredients | Percentage by weight III | IV | V | VI |
|---|---|---|---|---|
| **Surfactant** | | | | |
| Linear alkylbenzene sulfonate | 8 | 8 | 8 | 8 |
| Tallow alkyl sulphate | 2 | 2 | 2 | 2 |
| $C_{12}$-$C_{14}$ dimeth. hydroxyeth ammonium chlo | 5 | - | 5 | - |
| Fatty alcohol ($C_{12}$-$C_{15}$) ethoxylate | 1.5 | 1.5 | 1.5 | 1.5 |
| **Builder/chelants** | | | | |
| Zeolite A | 18.5 | 18.5 | 18.5 | 18.5 |
| Copolymer of maleic and acrylic acid, sodium salt | 5 | 5 | 5 | 5 |
| **Bleach** | | | | |
| Sodium perborate | 11 | 11 | 11 | 11 |
| N,N,N,T-Tetraacetyl ethylene diamine | 4 | 4 | 4 | 4 |
| **Perfume** | 0.5 | 0.5 | 0.5 | 0.5 |
| Enzymes | - | - | - | - |
| Protease | 1.6 | 1.6 | 1.6 | 1.6 |
| Cellulase 43 kD | 0.5 | 0.5 | - | - |
| **Softness system** | | | | |
| Smectite/montmorillonite clays | 12.5 | 12.5 | 12.5 | 12.5 |
| Polyethylene oxide | 0.3 | 0.3 | 0.3 | 0.3 |
| **Buffer** | | | | |
| Carbonate | 10.6 | 10.6 | 10.6 | 10.6 |
| Silicate (2.0) | 4 | 4 | 4 | 4 |
| CMC, chelants | | | | |
| Admix and spray-on (suds suppression, miscellaneous,...) | | balance to 100 | | |

Results :

Detergent composition III 0.5% (= 40 x $10^{-6}$% mg proteine wash liquor) 43 kD cellulase + 5% quaternary ammonium component versus detergent composition IV 0.5% (= 40 x $10^{-6}$% mg proteine wash liquor) 43 kD cellulase no quaternary ammonium component

| Cycles | 1 | 4 | 8 |
|---|---|---|---|
| PSU | 0 | 0.8s | 1.8s |

Detergent composition V + 5% quaternary ammonium composition versus detergent composition VI no quaternary ammonium component

| Cycles | 1 | 4 | 8 |
|---|---|---|---|
| PSU | - | - | 0.1 |

significant difference at 95% confidence

Conclusion :

The above results demonstate that the quaternary ammonium compound / 43 kD cellulase combination gives a statistical significant better performance than the sum of the individual actions of both ingredients.

Formulation examples :

The following compositions are made :

| Ingredients | Composition (% by weight) | | | |
|---|---|---|---|---|
| | Regular products | | | |
| | I | II | III | IV |
| $C_{11-12}$ alkyl benzene sulfonate | 7 | 5 | 4 | - |
| Tallow alcohol sulfate (Na) | - | 2 | - | - |
| $C_{14-15}$ alkyl sulfate (Na) | - | - | 3 | 4 |
| A-Olefin ($C_{12-18}$) sulfonate (Na) | - | - | - | 0.5 |
| Tallow alcohol ethoxylate ($EO_{11}$) | 0.5 | - | - | - |
| Fatty alcohol ($C_{12-15}$) ethoxylate ($EO_7$) | - | - | - | 0.5 |
| Hydrogenated tallow fatty acid | - | 0.5 | - | - |
| $C_{12-14}$ Dimethyl (hydroethyl) ammonium chloride | - | - | 5 | 5 |
| Sodium tripolyphosphate | 24 | - | - | 25 |
| Zeolite A | - | 20 | 20 | - |
| Sodium citrate | - | 5 | 5 | - |
| Oleic fatty acid | - | - | - | - |
| Citric acid | - | - | - | - |
| $C_{14-16}$ alkyl succinate | - | - | - | - |
| 1,2-Propanediol | - | - | - | - |
| Ethanol | - | - | - | - |
| Na Metaborate Octahydrate | - | - | - | - |
| Polyethylene oxide 5MM molecular weight | 0.05 | - | - | 0.05 |
| Polyethylene oxide 0.3MM molecular weight | - | 0.3 | - | - |
| Sodium sulfate | 12 | 10 | 15 | 5 |
| Sodium carbonate | 5 | 7 | - | 15 |
| Sodium silicate | 4 | 4 | 4 | 4 |
| Sodium perborate (1 aq.) | 15 | 15 | 18 | 15 |
| N,N,N,N-Tetraacetylethylene diamine | 3 | 3 | - | 3 |
| CMC | 0.3 | 0.3 | 0.3 | 0.3 |
| Polyacrylate (MW 1000-20 000) | - | 1.5 | - | - |
| Polyacrylate (MW 4000-5000) | - | - | 3 | - |
| Maleic-acrylic copolymer | 2 | - | - | 3 |
| Cellulase | 0.5 | 0.5 | 0.5 | 1 |
| Smectite/montmorillonite clay | 10.5 | 10.5 | 10.5 | 10.5 |
| Phosphate | - | - | - | 0.3 |
| Admix and spray-on (perfumes, protease, amylase, lipolase, buffer, sud suppression, miscelaneous, moisture and minors) | balance to 100 | | | |

| Ingredients | Composition (% by weight) | | |
|---|---|---|---|
| | Compact product | | Liquid product |
| | I | II | I |
| $C_{11-12}$ alkyl benzene sulfonate | 8 | - | 10 |
| Tallow alcohol sulfate (Na) | 2 | 2 | - |
| $C_{14-15}$ alkyl sulfate (Na) | - | 6 | 1 |
| A-Olefin ($C_{12-18}$) sulfonate (Na) | - | - | - |
| Tallow alcohol ethoxylate ($EO_{11}$) | - | - | - |
| Fatty alcohol ($C_{12-15}$) ethoxylate ($EO_7$) | - | - | - |
| Hydrogenated tallow fatty acid | - | - | - |
| $C_{12-14}$ Dimethyl (hydroethyl) ammonium chloride | 5 | 5 | - |
| Sodium tripolyphosphate | - | - | - |
| Zeolite A | 15 | 19 | - |
| Sodium citrate | - | 6 | - |
| Oleic fatty acid | - | - | 1 |
| Citric acid | - | - | 2 |
| $C_{14-16}$ alkyl succinate | - | - | 10 |
| 1,2-Propanediol | - | - | 3 |
| Ethanol | - | - | 7 |
| Na Metaborate Octahydrate | - | - | 1 |
| Polyethylene oxide 5MM molecular weight | - | - | - |
| Polyethylene oxide 0.3MM molecular weight | 0.3 | 0.3 | - |
| sulfonate | - | 2 | - |
| Sodium carbonate | 11 | 11 | - |
| Sodium silicate | 4 | 3 | - |
| Sodium perborate (1 aq.) | 11 | 12 | - |
| N,N,N,N-Tetraacetylethylene diamine | 4 | 3 | - |
| CMC | 0.4 | 0.3 | - |
| Polyacrylate (MW 1000-20 000) | - | - | - |
| Polyacrylate (MW 4000-5000) | - | - | - |
| Maleic-acrylic copolymer | 5 | 4 | - |
| Cellulase | 1 | 0.5 | 1 |
| Smectite/montmorillonite clay | 12 | 12 | 10.5 |
| Layered silicate | 7 | - | - |
| Admix and spray-on (perfumes, protease, amylase, lipolase, buffer, sud suppression, miscelaneous, moisture and minors) | balance to 100 | | |

**INFORMATION FOR SEQ ID NO 1 :**

(i) SEQUENCE CHARACTERISTICS

(A) Length : 1060 base pairs
(B) Type : nucleic acid
(C) Strandedness : single
(D) Topology : linear

(ii) MOLECULE TYPE : cDNA

(iii) HYPOTHETICAL : NO

(iv) ORIGINAL SOURCE

(A) Organism : Humicola insolens
(B) Strain : DSM 1800

(ix) FEATURE

(A) Name/Key : mat_peptide
(B) Location : 73..927

(ix) FEATURE

(A) Name/Key : sig_peptide
(B) Location : 10..72

(ix) FEATURE

(A) Name/Key : CDS
(B) Location : 10..927

**INFORMATION FOR SEQ ID NO 2 :**

(i) SEQUENCE CHARACTERISTICS

(A) Length : 305 amino acids
(B) Type : amino acid
(D) Topology : linear

(ii) MOLECULE TYPE : protein

**INFORMATION FOR SEQ ID NO 3**

(i) SEQUENCE CHARACTERISTICS

(A) LENGTH : 1473 base pairs
(B) TYPE : nucleic acid
(C) STRANDEDNESS : single
(D) TOPOLOGY : linear

(ii) MOLECULE TYPE : cDNA

(iii) HYPOTHETICAL : NO

(iv) ANTI-SENSE : NO

(vi) ORIGINAL SOURCE

(A) ORGANISM : fusarium oxysporum
(B) STRAIN : DSM 2672

(ix) FEATURE

(A) NAME/KEY : CDS
(B) LOCATION : 97..1224

**INFORMATION FOR SEQ ID NO 4**

(i) SEQUENCE CHARACTERISTICS

(A) LENGTH : 376 amino acids
(B) TYPE : amino acid
(D) TOPOLOGY : linear

(ii) MOLECULE TYPE : protein

SEQUENCE DESCRIPTION: SEQ ID NO:1:

```
GGATCCAAG ATG CGT TCC TCC CCC CTC CTC CCG TCC GCC GTT GTG GCC        48
          Met Arg Ser Ser Pro Leu Leu Pro Ser Ala Val Val Ala
          -21 -20             -15                 -10

GCC CTG CCG GTG TTG GCC CTT GCC GCT GAT GGC AGG TCC ACC CGC TAC      96
Ala Leu Pro Val Leu Ala Leu Ala Ala Asp Gly Arg Ser Thr Arg Tyr
             -5                   1               5

TGG GAC TGC TGC AAG CCT TCG TGC GGC TGG GCC AAG AAG GCT CCC GTG     144
Trp Asp Cys Cys Lys Pro Ser Cys Gly Trp Ala Lys Lys Ala Pro Val
     10                  15                  20

AAC CAG CCT GTC TTT TCC TGC AAC GCC AAC TTC CAG CGT ATC ACG GAC     192
Asn Gln Pro Val Phe Ser Cys Asn Ala Asn Phe Gln Arg Ile Thr Asp
 25                  30                  35                  40

TTC GAC GCC AAG TCC GGC TGC GAG CCG GGC GGT GTC GCC TAC TCG TGC     240
Phe Asp Ala Lys Ser Gly Cys Glu Pro Gly Gly Val Ala Tyr Ser Cys
                 45                  50                  55

GCC GAC CAG ACC CCA TGG GCT GTG AAC GAC GAC TTC GCG CTC GGT TTT     288
Ala Asp Gln Thr Pro Trp Ala Val Asn Asp Asp Phe Ala Leu Gly Phe
             60                  65                  70

GCT GCC ACC TCT ATT GCC GGC AGC AAT GAG GCG GGC TGG TGC TGC GCC     336
Ala Ala Thr Ser Ile Ala Gly Ser Asn Glu Ala Gly Trp Cys Cys Ala
         75                  80                  85

TGC TAC GAG CTC ACC TTC ACA TCC GGT CCT GTT GCT GGC AAG AAG ATG     384
Cys Tyr Glu Leu Thr Phe Thr Ser Gly Pro Val Ala Gly Lys Lys Met
     90                  95                 100

GTC GTC CAG TCC ACC AGC ACT GGC GGT GAT CTT GGC AGC AAC CAC TTC     432
Val Val Gln Ser Thr Ser Thr Gly Gly Asp Leu Gly Ser Asn His Phe
105                 110                 115                 120

GAT CTC AAC ATC CCC GGC GGC GGC GTC GGC ATC TTC GAC GGA TGC ACT     480
Asp Leu Asn Ile Pro Gly Gly Gly Val Gly Ile Phe Asp Gly Cys Thr
                125                 130                 135
```

```
CCC CAG TTC GGC GGT CTG CCC GGC CAG CGC TAC GGC GGC ATC TCG TCC      528
Pro Gln Phe Gly Gly Leu Pro Gly Gln Arg Tyr Gly Gly Ile Ser Ser
            140                 145                 150

CGC AAC GAG TGC GAT CGG TTC CCC GAC GCC CTC AAG CCC GGC TGC TAC      576
Arg Asn Glu Cys Asp Arg Phe Pro Asp Ala Leu Lys Pro Gly Cys Tyr
        155                 160                 165

TGG CGC TTC GAC TGG TTC AAG AAC GCC GAC AAT CCG AGC TTC AGC TTC      624
Trp Arg Phe Asp Trp Phe Lys Asn Ala Asp Asn Pro Ser Phe Ser Phe
    170                 175                 180

CGT CAG GTC CAG TGC CCA GCC GAG CTC GTC GCT CGC ACC GGA TGC CGC      672
Arg Gln Val Gln Cys Pro Ala Glu Leu Val Ala Arg Thr Gly Cys Arg
185                 190                 195                 200

CGC AAC GAC GAC GGC AAC TTC CCT GCC GTC CAG ATC CCC TCC AGC AGC      720
Arg Asn Asp Asp Gly Asn Phe Pro Ala Val Gln Ile Pro Ser Ser Ser
                205                 210                 215

ACC AGC TCT CCG GTC AAC CAG CCT ACC AGC ACC AGC ACC ACG TCC ACC      768
Thr Ser Ser Pro Val Asn Gln Pro Thr Ser Thr Ser Thr Thr Ser Thr
            220                 225                 230

TCC ACC ACC TCG AGC CCG CCA GTC CAG CCT ACG ACT CCC AGC GGC TGC      816
Ser Thr Thr Ser Ser Pro Pro Val Gln Pro Thr Thr Pro Ser Gly Cys
        235                 240                 245

ACT GCT GAG AGG TGG GCT CAG TGC GGC GGC AAT GGC TGG AGC GGC TGC      864
Thr Ala Glu Arg Trp Ala Gln Cys Gly Gly Asn Gly Trp Ser Gly Cys
    250                 255                 260

ACC ACC TGC GTC GCT GGC AGC ACT TGC ACG AAG ATT AAT GAC TGG TAC      912
Thr Thr Cys Val Ala Gly Ser Thr Cys Thr Lys Ile Asn Asp Trp Tyr
265                 270                 275                 280

CAT CAG TGC CTG TAGACGCAGG GCAGCTTGAG GGCCTTACTG GTGGCCGCAA          964
His Gln Cys Leu
            285

CGAAATGACA CTCCCAATCA CTGTATTAGT TCTTGTACAT AATTTCGTCA TCCCTCCAGG    1024

GATTGTCACA TAAATGCAAT GAGGAACAAT GAGTAC                              1060
```

SEQUENCE DESCRIPTION: SEQ ID NO:2:

```
Met Arg Ser Ser Pro Leu Leu Pro Ser Ala Val Val Ala Ala Leu Pro
-21 -20                 -15                 -10

Val Leu Ala Leu Ala Ala Asp Gly Arg Ser Thr Arg Tyr Trp Asp Cys
 -5                   1               5                  10

Cys Lys Pro Ser Cys Gly Trp Ala Lys Lys Ala Pro Val Asn Gln Pro
            15                  20                  25

Val Phe Ser Cys Asn Ala Asn Phe Gln Arg Ile Thr Asp Phe Asp Ala
        30                  35                  40

Lys Ser Gly Cys Glu Pro Gly Gly Val Ala Tyr Ser Cys Ala Asp Gln
    45                  50                  55

Thr Pro Trp Ala Val Asn Asp Asp Phe Ala Leu Gly Phe Ala Ala Thr
60                  65                  70                  75

Ser Ile Ala Gly Ser Asn Glu Ala Gly Trp Cys Cys Ala Cys Tyr Glu
                80                  85                  90

Leu Thr Phe Thr Ser Gly Pro Val Ala Gly Lys Lys Met Val Val Gln
            95                  100                 105

Ser Thr Ser Thr Gly Gly Asp Leu Gly Ser Asn His Phe Asp Leu Asn
        110                 115                 120

Ile Pro Gly Gly Gly Val Gly Ile Phe Asp Gly Cys Thr Pro Gln Phe
    125                 130                 135

Gly Gly Leu Pro Gly Gln Arg Tyr Gly Gly Ile Ser Ser Arg Asn Glu
140                 145                 150                 155

Cys Asp Arg Phe Pro Asp Ala Leu Lys Pro Gly Cys Tyr Trp Arg Phe
                160                 165                 170

Asp Trp Phe Lys Asn Ala Asp Asn Pro Ser Phe Ser Phe Arg Gln Val
            175                 180                 185

Gln Cys Pro Ala Glu Leu Val Ala Arg Thr Gly Cys Arg Arg Asn Asp
        190                 195                 200

Asp Gly Asn Phe Pro Ala Val Gln Ile Pro Ser Ser Ser Thr Ser Ser
    205                 210                 215
```

```
Pro Val Asn Gln Pro Thr Ser Thr Ser Thr Thr Ser Thr Ser Thr Thr
220                 225                 230                 235

Ser Ser Pro Pro Val Gln Pro Thr Thr Pro Ser Gly Cys Thr Ala Glu
                240                 245                 250

Arg Trp Ala Gln Cys Gly Gly Asn Gly Trp Ser Gly Cys Thr Thr Cys
            255                 260                 265

Val Ala Gly Ser Thr Cys Thr Lys Ile Asn Asp Trp Tyr His Gln Cys
        270                 275                 280

Leu
```

SEQUENCE DESCRIPTION: SEQ ID NO:3:

```
GAATTCGCGG CCGCTCATTC ACTTCATTCA TTCTTTAGAA TTACATACAC TCTCTTTCAA        60

AACAGTCACT CTTTAAACAA AACAACTTTT GCAACA ATG CGA TCT TAC ACT CTT         114
                                        Met Arg Ser Tyr Thr Leu
                                          1               5

CTC GCC CTG GCC GGC CCT CTC GCC GTG AGT GCT GCT TCT GGA AGC GGT         162
Leu Ala Leu Ala Gly Pro Leu Ala Val Ser Ala Ala Ser Gly Ser Gly
             10                  15                  20

CAC TCT ACT CGA TAC TGG GAT TGC TGC AAG CCT TCT TGC TCT TGG AGC         210
His Ser Thr Arg Tyr Trp Asp Cys Cys Lys Pro Ser Cys Ser Trp Ser
         25                  30                  35

GGA AAG GCT GCT GTC AAC GCC CCT GCT TTA ACT TGT GAT AAG AAC GAC         258
Gly Lys Ala Ala Val Asn Ala Pro Ala Leu Thr Cys Asp Lys Asn Asp
     40                  45                  50

AAC CCC ATT TCC AAC ACC AAT GCT GTC AAC GGT TGT GAG GGT GGT GGT         306
Asn Pro Ile Ser Asn Thr Asn Ala Val Asn Gly Cys Glu Gly Gly Gly
 55                  60                  65                  70

TCT GCT TAT GCT TGC ACC AAC TAC TCT CCC TGG GCT GTC AAC GAT GAG         354
Ser Ala Tyr Ala Cys Thr Asn Tyr Ser Pro Trp Ala Val Asn Asp Glu
                 75                  80                  85

CTT GCC TAC GGT TTC GCT GCT ACC AAG ATC TCC GGT GGC TCC GAG GCC         402
Leu Ala Tyr Gly Phe Ala Ala Thr Lys Ile Ser Gly Gly Ser Glu Ala
             90                  95                 100
```

AGC TGG TGC TGT GCT TGC TAT GCT TTG ACC TTC ACC ACT GGC CCC GTC 450
Ser Trp Cys Cys Ala Cys Tyr Ala Leu Thr Phe Thr Thr Gly Pro Val
105 110 115

AAG GGC AAG AAG ATG ATC GTC CAG TCC ACC AAC ACT GGA GGT GAT CTC 498
Lys Gly Lys Lys Met Ile Val Gln Ser Thr Asn Thr Gly Gly Asp Leu
120 125 130

GGC GAC AAC CAC TTC GAT CTC ATG ATG CCC GGC GGT GGT GTC GGT ATC 546
Gly Asp Asn His Phe Asp Leu Met Met Pro Gly Gly Gly Val Gly Ile
135 140 145 150

TTC GAC GGC TGC ACC TCT GAG TTC GGC AAG GCT CTC GGC GGT GCC CAG 594
Phe Asp Gly Cys Thr Ser Glu Phe Gly Lys Ala Leu Gly Gly Ala Gln
155 160 165

TAC GGC GGT ATC TCC TCC CGA AGC GAA TGT GAT AGC TAC CCC GAG CTT 642
Tyr Gly Gly Ile Ser Ser Arg Ser Glu Cys Asp Ser Tyr Pro Glu Leu
170 175 180

CTC AAG GAC GGT TGC CAC TGG CGA TTC GAC TGG TTC GAG AAC GCC GAC 690
Leu Lys Asp Gly Cys His Trp Arg Phe Asp Trp Phe Glu Asn Ala Asp
185 190 195

AAC CCT GAC TTC ACC TTT GAG CAG GTT CAG TGC CCC AAG GCT CTC CTC 738
Asn Pro Asp Phe Thr Phe Glu Gln Val Gln Cys Pro Lys Ala Leu Leu
200 205 210

GAC ATC AGT GGA TGC AAG CGT GAT GAC GAC TCC AGC TTC CCT GCC TTC 786
Asp Ile Ser Gly Cys Lys Arg Asp Asp Asp Ser Ser Phe Pro Ala Phe
215 220 225 230

AAG GTT GAT ACC TCG GCC AGC AAG CCC CAG CCC TCC AGC TCC GCT AAG 834
Lys Val Asp Thr Ser Ala Ser Lys Pro Gln Pro Ser Ser Ser Ala Lys
235 240 245

AAG ACC ACC TCC GCT GCT GCT GCC GCT CAG CCC CAG AAG ACC AAG GAT 882
Lys Thr Thr Ser Ala Ala Ala Ala Ala Gln Pro Gln Lys Thr Lys Asp
250 255 260

TCC GCT CCT GTT GTC CAG AAG TCC TCC ACC AAG CCT GCC GCT CAG CCC 930
Ser Ala Pro Val Val Gln Lys Ser Ser Thr Lys Pro Ala Ala Gln Pro
265 270 275

GAG CCT ACT AAG CCC GCC GAC AAG CCC CAG ACC GAC AAG CCT GTC GCC 978
Glu Pro Thr Lys Pro Ala Asp Lys Pro Gln Thr Asp Lys Pro Val Ala
280 285 290

ACC AAG CCT GCT GCT ACC AAG CCC GTC CAA CCT GTC AAC AAG CCC AAG 1026
Thr Lys Pro Ala Ala Thr Lys Pro Val Gln Pro Val Asn Lys Pro Lys
295 300 305 310

ACA ACC CAG AAG GTC CGT GGA ACC AAA ACC CGA GGA AGC TGC CCG GCC 1074
Thr Thr Gln Lys Val Arg Gly Thr Lys Thr Arg Gly Ser Cys Pro Ala
315 320 325

```
AAG ACT GAC GCT ACC GCC AAG GCC TCC GTT GTC CCT GCT TAT TAC CAG        1122
Lys Thr Asp Ala Thr Ala Lys Ala Ser Val Val Pro Ala Tyr Tyr Gln
            330                 335                 340

TGT GGT GGT TCC AAG TCC GCT TAT CCC AAC GGC AAC CTC GCT TGC GCT        1170
Cys Gly Gly Ser Lys Ser Ala Tyr Pro Asn Gly Asn Leu Ala Cys Ala
        345                 350                 355

ACT GGA AGC AAG TGT GTC AAG CAG AAC GAG TAC TAC TCC CAG TGT GTC        1218
Thr Gly Ser Lys Cys Val Lys Gln Asn Glu Tyr Tyr Ser Gln Cys Val
    360                 365                 370

CCC AAC TAAATGGTAG ATCCATCGGT TGTGGAAGAG ACTATGCGTC TCAGAAGGGA         1274
Pro Asn
375

TCCTCTCATG AGCAGGCTTG TCATTGTATA GCATGGCATC CTGGACCAAG TGTTCGACCC      1334

TTGTTGTACA TAGTATATCT TCATTGTATA TATTTAGACA CATAGATAGC CTCTTGTCAG      1394

CGACAACTGG CTACAAAAGA CTTGGCAGGC TTGTTCAATA TTGACACAGT TTCCTCCATA      1454

AAAAAAAAAA AAAAAAAAA                                                   1473
```

SEQUENCE DESCRIPTION: SEQ ID NO:4:

```
Met Arg Ser Tyr Thr Leu Leu Ala Leu Ala Gly Pro Leu Ala Val Ser
 1               5                  10                  15

Ala Ala Ser Gly Ser Gly His Ser Thr Arg Tyr Trp Asp Cys Cys Lys
            20                  25                  30

Pro Ser Cys Ser Trp Ser Gly Lys Ala Ala Val Asn Ala Pro Ala Leu
        35                  40                  45

Thr Cys Asp Lys Asn Asp Asn Pro Ile Ser Asn Thr Asn Ala Val Asn
    50                  55                  60

Gly Cys Glu Gly Gly Gly Ser Ala Tyr Ala Cys Thr Asn Tyr Ser Pro
65                  70                  75                  80

Trp Ala Val Asn Asp Glu Leu Ala Tyr Gly Phe Ala Ala Thr Lys Ile
                85                  90                  95

Ser Gly Gly Ser Glu Ala Ser Trp Cys Cys Ala Cys Tyr Ala Leu Thr
            100                 105                 110

Phe Thr Thr Gly Pro Val Lys Gly Lys Lys Met Ile Val Gln Ser Thr
        115                 120                 125

Asn Thr Gly Gly Asp Leu Gly Asp Asn His Phe Asp Leu Met Met Pro
    130                 135                 140

Gly Gly Gly Val Gly Ile Phe Asp Gly Cys Thr Ser Glu Phe Gly Lys
145                 150                 155                 160

Ala Leu Gly Gly Ala Gln Tyr Gly Gly Ile Ser Ser Arg Ser Glu Cys
                165                 170                 175

Asp Ser Tyr Pro Glu Leu Leu Lys Asp Gly Cys His Trp Arg Phe Asp
            180                 185                 190

Trp Phe Glu Asn Ala Asp Asn Pro Asp Phe Thr Phe Glu Gln Val Gln
        195                 200                 205

Cys Pro Lys Ala Leu Leu Asp Ile Ser Gly Cys Lys Arg Asp Asp Asp
    210                 215                 220

Ser Ser Phe Pro Ala Phe Lys Val Asp Thr Ser Ala Ser Lys Pro Gln
225                 230                 235                 240
```

Pro Ser Ser Ser Ala Lys Lys Thr Thr Ser Ala Ala Ala Ala Ala Gln
245 250 255

Pro Gln Lys Thr Lys Asp Ser Ala Pro Val Val Gln Lys Ser Ser Thr
260 265 270

Lys Pro Ala Ala Gln Pro Glu Pro Thr Lys Pro Ala Asp Lys Pro Gln
275 280 285

Thr Asp Lys Pro Val Ala Thr Lys Pro Ala Ala Thr Lys Pro Val Gln
290 295 300

Pro Val Asn Lys Pro Lys Thr Thr Gln Lys Val Arg Gly Thr Lys Thr
305 310 315 320

Arg Gly Ser Cys Pro Ala Lys Thr Asp Ala Thr Ala Lys Ala Ser Val
325 330 335

Val Pro Ala Tyr Tyr Gln Cys Gly Gly Ser Lys Ser Ala Tyr Pro Asn
340 345 350

Gly Asn Leu Ala Cys Ala Thr Gly Ser Lys Cys Val Lys Gln Asn Glu
355 360 365

Tyr Tyr Ser Gln Cys Val Pro Asn
370 375

## Claims

1. A detergent composition comprising a quaternary ammonium compound of the formula

$R_1R_2R_3R_4N^+ X^-$

wherein $R_1$ is $C_8$-$C_{16}$ alkyl, each of $R_2$, $R_3$ and $R_4$ is independently $C_1$-$C_4$ alkyl or hydroxyl alkyl, benzyl, or $-(C_2H_4O)_xH$ where x has a value from 2 to 5, not more than one of $R_2$, $R_3$ or $R_4$ being benzyl and $X^-$ is an anion and a cellulase, characterized in that the cellulase provides at least 10% removal of immobilized radioactive labelled carboxymethyl cellulose according to the C14CMC method at 25 x $10^{-6}$% by weight of the cellulase protein in the laundry test solution.

2. A detergent composition according to claim 1 wherein the cellulase consists essentially of a homogeneous endoglucanase component which is immunoreactive with an antibody raised against a highly purified about 43 kD cellulase derived from Humicola insolens, DSM 1800, or which is homologous to said 43kD endoglucanase.

3. A detergent composition according to claim 2 wherein the endoglucanase component of said cellulase has an isoelectric point of about 5.1.

4. A detergent composition according to claims 2 or 3 wherein said endoglucanase component is producible by a method comprising cultivating a host cell transformed with a recombinant DNA vector carrying a DNA sequence encoding said endoglucanase component or a precursor of said endoglucanase component, as well as DNA sequences encoding functions permitting the expression of the DNA sequence encoding the endoglucanase component, or a precursor thereof, in a culture medium under conditions permitting the expression of the endoglucanase component or precursor thereof and recovering the endoglucanase component form the culture.

5. A detergent composition according to claim 1 wherein the cellulase has the amino acid sequence

shown in the appended sequence listing ID#2, or is a homologue thereof exhibiting endoglucanase activity.

6. A detergent composition according to claim 3 wherein said cellulase is producible by a species of Humicola, e.g. Humicola insolens.

7. A detergent composition according to claim 1 characterized in that the cellulase compound is and endoglucanase enzyme having the amino acid sequence shown in the appended sequence lising ID#4, or is a homologue thereof exhibiting endoglucanase activity.

8. A detergent composition according to claim 7 wherein said endoglucanase enzyme is producible by a species of Fusarium, e.g. Fusarium oxysporum.

9. A detergent composition according to any of the claims 5 to 8 wherein said enzyme is produced by a DNA construct comprising a DNA sequence encoding the enzyme.

10. A detergent composition according to claim 9 wherein the DNA sequence is as shown in the appended sequence listings ID#1 or ID#3.

11. A detergent composition according to any of the claims 5 to 10 wherein said host cell is a strain of a fungus such as Tricloderuca or Aspergillus, preferably Aspergillus oryzae or Aspergillus niger, or a yeqst cell belonging to a strain of Hansenula or Saccharomyces, e.g. a strain of Saccachomyces cerevisae.

12. A detergent composition according to any of the claims 5 to 10 wherein said host cell is a strain of a bacterium, e.g. Bacillus, Streptomyces or E. coli.

13. A detergent composition according to claims 1 to 12, wherein the water soluble cationic compound is selected from quaternary ammonium salts in which $R_1$ is $C_{12}$-$C_{14}$ alkyl and $R_2$, $R_3$ and $R_4$ are selected from methyl and hydroxyethyl groups.

14. A detergent composition according to claims 1-13, wherein the level of said watersoluble cationic compound is from 0.2 to 10%.

15. A detergent composition according to claims 1-14, further comprising anionic surfactants, wherein the molar ratio of the anionic surfactant to the water soluble quaternary ammonium compound is greater than 1:1.

16. A detergent composition according to any of the preceding claims, which is a detergent additive, in the form of a non-dusting granulate or liquid.

17. A detergent composition which comprises a detergent additive according to any of claims 1-16.

18. A detergent composition according to claim 17 which is in granular form, compact granular form or liquid form.

19. A laundry process comprising contacting fabrics with a detergent composition according to claims 1-18, wherein the cellulase being added at levels of from 0.005 to 40 mg enzyme protein/liter of wash solution.

European Patent Office

# EUROPEAN SEARCH REPORT

Application Number

EP 92 20 0101

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | RESEARCH DISCLOSURE. vol. 292, August 1988, NEW YORK US pages 598 - 603; 'Cellulase in fabric washing compositions' * page 602, right column, paragraph 4 -paragraph 5 * | 1 | C11D3/386 C12N9/42 C12N15/56 C11D17/06 C11D1/62 |
| A | | 13-17 | |
| D,A | WO-A-8 909 259 (NOVO INDUSTRI A/S) * abstract; claims 1-25 * | 1,2,6,8, 9,11,12, 19 | |
| D,A | EP-A-0 350 098 (PROCTER & GAMBLE COMPANY) | 1 | |
| A | EP-A-0 381 397 (UNILEVER PLC) * page 6, line 35 - line 46; claims 1-15 * | 1,18 | |
| A | EP-A-0 173 397 (UNILEVER NV) * abstract; claims 1,5,6 * | 1,13-15, 19 | **TECHNICAL FIELDS SEARCHED (Int. Cl.5 )** |
| A | EP-A-0 095 205 (THE PROCTER & GAMBLE CO.) * abstract; claims 1,6,11 * | 1,13-15, 19 | C11D C12N |
| A | WORLD PATENTS INDEX LATEST Week 8807, Derwent Publications Ltd., London, GB; AN 88-047365 & JP-A-63 006 098 (LION CORP) 12 January 1988 * abstract * | 1,13 | |
| A | EP-A-0 390 446 (ECC INTERNATIONAL LTD.) * abstract; claims 1-5 * | 1,14,15 | |

The present search report has been drawn up for all claims

| Place of search | Date of completion of the search | Examiner |
|---|---|---|
| BERLIN | 19 MARCH 1992 | GURDJIAN D. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document